(19) Europäisches Patentamt
European Patent Office
Office européen des brevets

(11) **EP 3 991 705 A2**

(12) **EUROPEAN PATENT APPLICATION**
published in accordance with Art. 153(4) EPC

(43) Date of publication:
**04.05.2022 Bulletin 2022/18**

(21) Application number: **20830691.0**

(22) Date of filing: **25.06.2020**

(51) International Patent Classification (IPC):
*A61F 11/00* (2022.01)      *A61B 5/00* (2006.01)
*A61B 3/113* (2006.01)      *A61B 5/16* (2006.01)
*A61B 5/11* (2006.01)       *A63F 13/65* (2014.01)
*G02B 27/01* (2006.01)      *G16H 20/70* (2018.01)

(52) Cooperative Patent Classification (CPC):
**A61B 3/113; A61B 5/00; A61B 5/11; A61B 5/16;
A61F 11/00; A63F 13/65; G02B 27/01; G16H 20/70**

(86) International application number:
**PCT/KR2020/008255**

(87) International publication number:
**WO 2020/262973 (30.12.2020 Gazette 2020/53)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.06.2019   KR 20190075599
23.09.2019   KR 20190116774**

(71) Applicant: **Korea University Research and
Business Foundation
Seoul 02841 (KR)**

(72) Inventor: **RAH, Yoon Chan
Seoul 06547 (KR)**

(74) Representative: **Roos, Peter
c/o Roospatent
Noldinstrasse 7
81545 München (DE)**

(54) **VIRTUAL REALITY GAME- AND BIOSIGNAL SENSOR-BASED VESTIBULO-OCULAR REFLEX ASSESSMENT AND REHABILITATION APPARATUS**

(57)    The present invention relates to a virtual reality-based vestibulo-ocular reflex (VOR) assessment and rehabilitation apparatus. The virtual reality game- and biosignal sensor-based VOR assessment and rehabilitation apparatus may comprise: a biosignal sensing unit which senses and notifies of head movement and eye movement of a patient using an eye tracker and an inertial measurement unit (IMU) sensor attached or installed to a head-mounted display device; a VOR function assessment unit which, when a VOR function assessment is requested, configures and provides a game for VOR function assessment, and then obtains a VOR gain by calculating and quantifying the ratio of eye to head movement from the sensing result of the IMU sensor and the eye tracker; a patient-personalization control unit which, when rehabilitation exercise is requested, determines, on the basis of the VOR gain of the patient, the types and order of rehabilitation games, and a rehabilitation level; and a rehabilitation game providing unit which, according to the types and order of the rehabilitation games, configures and provides games for at least one among smooth pursuit, saccades, VOR rehabilitation exercise, amplified VOR rehabilitation exercise, and suppressive VOR rehabilitation exercise, and determines the difficulty levels of the games on the basis of the rehabilitation level.

FIG. 1

**Description**

TECHNICAL FIELD

The present invention relates to a virtual reality game- and biosignal sensor-based vestibulo-ocular reflex assessment and rehabilitation apparatus in which a vestibulo-ocular reflex function is assessed by using a head-mounted display device, an eye tracker, and an inertial measurement sensor (IMU sensor), and rehabilitation exercise optimized for the vestibulo-ocular reflex function can be performed in a virtual reality game method.

BACKGROUND

In short, a vestibulo-ocular reflex (VOR), which has the highest clinical importance among vestibular functions, refers to a function that induces eye movement in the opposite direction to the rotation of the head. Through the VOR, animals, including humans, can keep an eye on a specific point or object regardless of the head movement.

In a case where a vestibular dysfunction, in which functions of vestibular organs located on both sides thereof are damaged due to various causes, occurs, an abnormality occurs in the VOR and the eye cannot rotate in the opposite direction thereto as the head rotates.

Such a vestibular dysfunction can be improved through a vestibular rehabilitation exercise configured of various eye and head rotation exercises. However, the conventional vestibular rehabilitation exercise is configured of a program that simply repeats operations such as eye rotation and head rotation, so interest is easily lost thereby having a disadvantage that an actual implementation rate is very low.

The conventional vestibular rehabilitation exercise has a limitation in that it is impossible to provide a patient-personalization treatment by configuring and providing the same program to patients regardless of a degree of a decrease in the vestibular function. In addition, since the same program is continuously provided to the patients without considering the vestibular function which is improved through rehabilitation training and a natural recovery process, there is a problem that the efficiency of treatment is lowered.

On the other hand, in order to measure the vestibular function, it is inconvenient for the patient to visit a hospital and perform an expensive vestibular function test. Since the vestibular function has to be measured by using a separate vestibular function measuring device, there is a limitation in that the vestibular function cannot be measured during the rehabilitation exercise.

In addition, in order to assess the improvement of the function of visual function training which is used as a sensory substitution method, since the assessment has to be performed by visiting the hospital and using an expensive existing measuring device, there is a limitation in that the improvement of visual function is not actually measured in the rehabilitation process and a large test cost has to be separately paid.

SUMMARY OF INVENTION

Technical Problem

Accordingly, the present invention is provided to solve the above problems and the present invention provides a virtual/augmented reality game- and biosignal sensor-based vestibulo-ocular reflex assessment and rehabilitation apparatus capable of assessing a VOR function of a patient in real time by using a head-mounted display device, an eye tracker, and an inertial measurement sensor (IMU sensor).

In addition, the present invention is intended to provide a virtual reality game- and biosignal sensor-based vestibulo-ocular reflex assessment and rehabilitation apparatus capable of performing a rehabilitation exercise optimized for a VOR function of a patient in a virtual reality game method.

In addition, the present invention is intended to provide a virtual reality game- and biosignal sensor-based vestibulo-ocular reflex assessment and rehabilitation apparatus capable of performing a swivel chair test operation by using a general swivel chair anytime and anywhere.

In addition, the present invention is intended to provide a central processing unit that calculates and outputs a test result and a personalized vestibular rehabilitation program based on a portable apparatus capable of a vestibular-ocular reflex test and rehabilitation, and measured data, and a platform that connects the portable apparatus and the central processing unit via an online network.

Objects of the present invention are not limited to the objects mentioned above, and other objects not mentioned will be clearly understood by those of ordinary skill in the art to which the present invention belongs from the following description.

Solution to Problem

As means for solving the problems described above, according to an embodiment of the present invention, there is provided a virtual reality game- and biosignal sensor-based vestibulo-ocular reflex assessment and rehabilitation apparatus, including: a biosignal sensing unit which senses and notifies of head movement and eye movement of a patient using an eye tracker and an inertial measurement unit (IMU) sensor attached or installed to a head-mounted display device; a VOR function assessment unit which, when a VOR function assessment is requested, configures and provides a game for VOR function assessment, and then obtains a VOR gain by calculating and quantifying the ratio of eye to head movement from the sensing result of the IMU sensor and the eye tracker; a patient-personalization control unit which, when rehabilitation exercise is requested, determines, on the basis of the VOR gain of the patient, the types and order of rehabilitation games, and a rehabilitation level; and a rehabilitation game providing unit which, according to the types and order of the rehabilitation games, configures and provides games for at least one of a smooth pursuit, a saccade, a VOR rehabilitation exercise, an amplified VOR rehabilitation exercise, and a suppressive VOR rehabilitation exercise, and determines the difficulty levels of the games on the basis of the rehabilitation level.

The patient-personalization control unit further includes a function of frequently adjusting the type and order of the rehabilitation game, and the rehabilitation level thereof in consideration of the game result of the rehabilitation game providing unit while the rehabilitation exercise is being performed.

The VOR function assessment unit configures and provides a game in which the eye observes the eye target on the screen and which induces a head rotation at a preset angle and velocity to induce the VOR.

The rehabilitation game providing unit configures and provides a game for eye-tracking an eye target in which the eye moves smoothly at a set speed when the smooth pursuit is requested, and then calculates and analyzes a ratio of the eye movement to the eye target to determine whether the game is cleared.

The rehabilitation game providing unit configures and provides a game for eye-tracking an eye target in which the eye momentarily moves from one place to another when the saccade is requested, and then calculates and analyzes a ratio of the eye movement to the eye target to determine whether the game is cleared.

The rehabilitation game providing unit configures and provides a game for rotating only the head at a preset angle and speed while keeping the eye fixed when the VOR rehabilitation exercise is requested, and then calculates and analyzes a ratio of the eye movement to the eye target to determine whether the game is cleared.

The rehabilitation game providing unit configures and provides a game for inducing the head movement at a preset angle and speed, and further rotating the eye target at a ratio or more to the head rotation to induce a greater range of the eye movement when the amplified VOR rehabilitation exercise is requested, and then calculates and analyzes a ratio of the eye movement to the eye target to determine whether the game is cleared.

The rehabilitation game providing unit configures and provides a game for inducing the head movement at a preset angle and speed, and inducing the eye movement for suppressing the eye target to be rotated less at a certain ratio to the head rotation when the suppressive VOR rehabilitation exercise is requested, and then calculates and analyzes a ratio of the eye movement to the eye target to determine whether the game is cleared.

The rehabilitation game providing unit further includes a function of monitoring the concentration of the patient, and additionally generating and providing information for evoking attention when the concentration falls to a preset value or more.

The rehabilitation game providing unit adjusts a game difficulty through at least one of the movement speed of the eye target, the rotation speed of the head, and the movement speed of the background screen.

In addition, the virtual reality game- and biosignal sensor-based vestibulo-ocular reflex assessment and rehabilitation apparatus further includes a swivel chair that supports seating and rotation of a testee.

The VOR function assessment unit further includes a function of generating a test screen for providing a dark field to reproduce the test screen via the head-mounted display device, and then calculating and quantifying the ratio of the eye rotation to the head rotation from the sensing results of the eye tracker and the inertial measurement sensor to calculate and provide at least one of the VOR (Vestibulo-Ocular Reflex) gain and the asymmetry ratio.

The VOR function assessment unit further includes a function of generating and providing a test screen inducing preset head rotation and eye rotation, and then calculating and quantifying the ratio of the eye rotation to the head rotation from the sensing results of the eye tracker and the inertial measurement sensor to calculate and provide at least one of the VOR (Vestibulo-Ocular Reflex) gain and the asymmetry ratio.

The VOR function assessment unit configures and provides a test screen inducing preset head rotation and eye rotation, and adjusts, in multiple steps, the ratio of the eye rotation to the head rotation, the rotation angles and speeds of the head and the eye.

The VOR function assessment unit calculates the rotation angle-based gain from a ratio of the eye rotation to the head rotation angle, calculates a maximum rotation angular velocity-based gain from a maximum eye rotation angular velocity to a maximum head rotation angular velocity, calculates a maximum rotation angular acceleration-based gain from a maximum eye rotation angular acceleration to a maximum head rotation angular acceleration, and then selects and provides, as the VOR gain, at least one of the rotation angle-based gain, the maximum rotation angular velocity-based gain, and the maximum rotation angular acceleration-based gain.

The VOR function assessment unit calculates the asymmetry ratio according to the expression "$\frac{(degree\ of\ rotation\ to\ right) - (degree\ of\ rotation\ to\ left)}{(degree\ of\ rotation\ to\ right) + (degree\ of\ rotation\ to\ left)}$", and the degree of the rotation is any one of the rotation angle, the rotation angular velocity, and the rotation angular acceleration.

The VOR function assessment unit requests a retry of the head rotation, and then performs the vestibulo-ocular reflex function assessment operation once more when the head rotation is not within a preset target frequency.

The swivel chair is automatically rotated under the control of the control device or manually rotated by the testee or the tester.

As means for solving the problems described above, according to another embodiment of the present invention, there is provided a virtual/ augmented reality-based vestibulo-ocular reflex assessment and rehabilitation system including: a plurality of vestibulo-ocular reflex assessment and rehabilitation apparatuses which reproduce a screen for a VOR (Vestibulo-Ocular Reflex) function assessment or a rehabilitation exercise, use an eye tracker and an inertial measurement sensor (IMU sensor) attached or installed on a head-mounted display device to sense and output a head movement and an eye movement of a patient; and a central control server which divides information provided by each of the vestibulo-ocular reflex assessment and rehabilitation apparatuses for each patient account to store the information in a database, and acquires and analyzes the patient information stored in the database to assess a VOR gain, or configures and provides a patient-personalization rehabilitation program based on the VOR gain. In the patient-personalization rehabilitation program, at least one of a smooth pursuit, a saccade, a VOR rehabilitation exercise, an amplified VOR rehabilitation exercise, and a suppressive VOR rehabilitation exercise is performed in a virtual reality game method.

Advantageous Effects

The present invention makes it possible to more simply and easily assess the vestibulo-ocular reflex function of an individual patient by using the head-mounted display device, the eye tracker, and the inertial measurement sensor (IMU sensor).

In addition, the vestibular rehabilitation exercise of the virtual reality game method can be performed, the game difficulty can be adjusted frequently according to the VOR function, thereby enabling a patient-personalization and motivational vestibular rehabilitation exercise.

In addition, by configuring the vestibulo-ocular reflex assessment and rehabilitation apparatus as a portable apparatus configured of an online network, it is possible to solve the temporal/spatial constraints of vestibular function test and rehabilitation.

BRIEF DESCRIPTION OF THE DRAWINGS

FIG. 1 is a diagram illustrating a virtual reality-based vestibulo-ocular reflex assessment and rehabilitation apparatus according to an embodiment of the present invention.

FIG. 2 is a diagram illustrating a detailed configuration of a biosignal sensing unit according to an embodiment of the present invention.

FIG. 3 is a diagram for explaining a VOR function assessment method according to an embodiment of the present invention.

FIG. 4 is a flowchart for explaining a rehabilitation exercise supporting method according to an embodiment of the present invention.

FIG. 5 is a diagram illustrating an example of a game screen for supporting a smooth pursuit according to an embodiment of the present invention.

FIG. 6 is a diagram illustrating an example of a game screen for supporting a saccade according to an embodiment of the present invention.

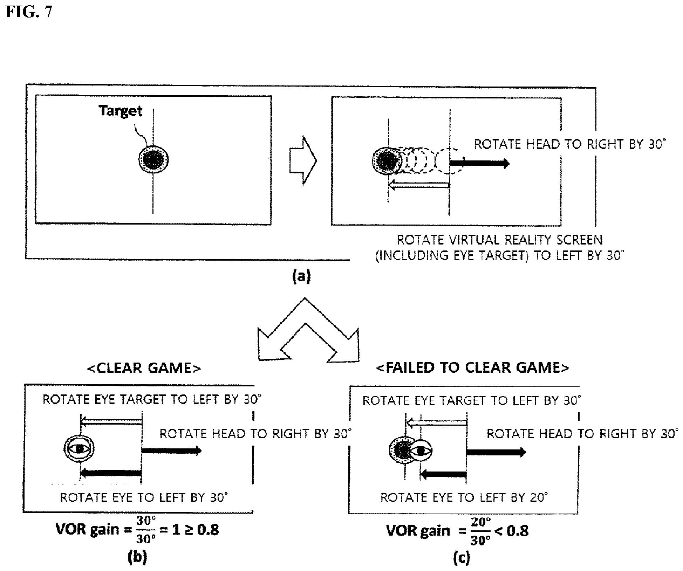

FIG. 7 is a diagram illustrating an example of a game screen for supporting a VOR rehabilitation exercise according to an embodiment of the present invention.

FIG. 8 is a diagram illustrating an example of a game screen for supporting an amplified VOR rehabilitation exercise according to an embodiment of the present invention.

FIG. 9 is a diagram illustrating an example of a game screen for supporting a suppressive VOR rehabilitation exercise according to an embodiment of the present invention.

FIG. 10 is a view for explaining a swivel chair-based vestibulo-ocular reflex assessment method.

FIG. 11 is a diagram illustrating a virtual reality-based vestibulo-ocular reflex assessment and rehabilitation apparatus according to another embodiment of the present invention.

FIG. 12 is a flowchart for explaining a vestibulo-ocular reflex function assessment method according to another embodiment of the present invention.

FIG. 14 is a diagram illustrating a virtual reality-based vestibulo-ocular reflex assessment and rehabilitation system according to another embodiment of the present invention.

BEST MODE FOR INVENTION

Objects and effects of the present invention, and technical configurations for achieving them will become clear with reference to the embodiments described below in detail in conjunction with the accompanying drawings. In describing the present invention, in a case where it is determined that a detailed description of a well-known function or configuration may unnecessarily obscure the gist of the present invention, the detailed description thereof will be omitted.

In addition, the terms to be described later are terms defined in consideration of functions in the present invention, which may vary depending on the intention or custom of a patient or an operator.

However, the present invention is not limited to the embodiments disclosed below and may be implemented in various different forms. Only the present embodiments are provided such that the disclosure of the present invention is complete and those of ordinary skill in the art to which the present invention belongs are fully informed. The present invention is defined only by the scope of the claims. Therefore, the definition thereof has to be made based on the contents throughout this specification.

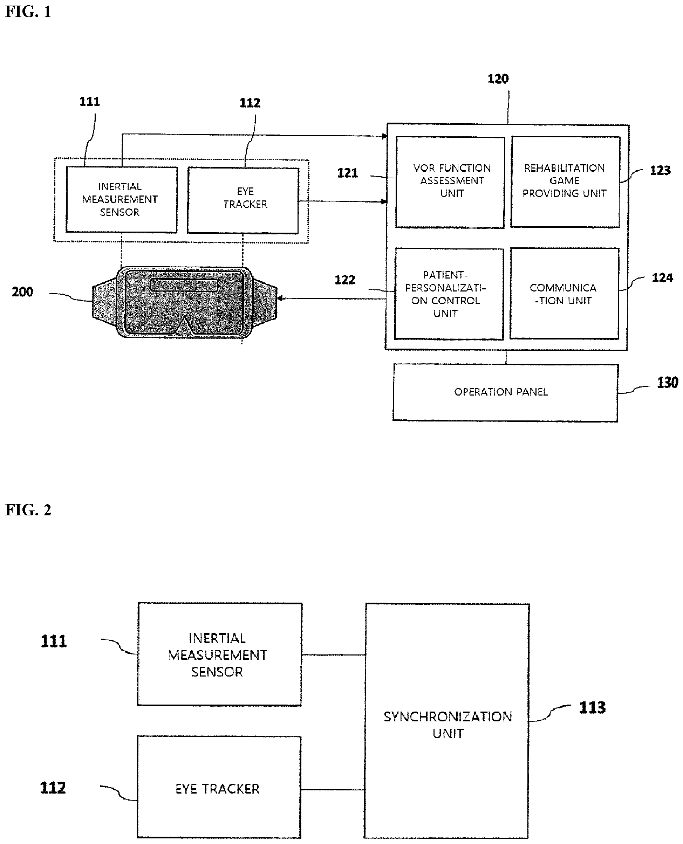

FIG. 1 is a diagram illustrating a virtual reality-based vestibulo-ocular reflex assessment and rehabilitation apparatus according to an embodiment of the present invention.

As illustrated in FIG. 1, a vestibulo-ocular reflex assessment and rehabilitation apparatus 100 of the present invention includes a biosignal sensing unit 110 that interoperates with a head-mounted display device 200 capable of reproducing an image in a virtual reality method, a control device 120, and an operation panel 130.

In this case, the head-mounted display device 200 reproduces various screens provided by the control device 120 in VR (virtual reality), AR (augmented reality), MR (mixed reality), XR (extended reality), and the like to allow a testee to experience a virtual image world as if it were a real world. This can be implemented by a head-mounted display (HMD), Google Glass, Microsoft Holo Lens, or the like, but is not limited thereto.

The biosignal sensing unit 110 includes an inertial measurement sensor 111 and an eye tracker 112 that are attached or installed on the head-mounted display device, and senses and outputs the head and eye movements of the patient through them.

The control device 120 includes a vestibulo-ocular reflex (VOR) assessment unit 121, a patient-personalization control unit 122, a rehabilitation game providing unit 123, a communication unit 124, and the like to support a VOR function assessment operation and various rehabilitation exercise support operations through them.

When the VOR function assessment is requested by the patient, the VOR function assessment unit 121 configures and provides a game that simultaneously induces the eye and head movements through a game screen in which an eye target is fixed at a center of the screen and only a background screen is rotated at a preset angle. Then, a VOR gain is acquired and stored by calculating and quantifying a ratio of the eye movement to the head movement based on a sensing result of the biosignal sensing unit 110.

For reference, during actual daily life, the head rotation and the eye movement occur at the same time, and the rehabilitation exercise, in which the head rotates and the eye target moves in various directions and speeds (angular velocity and angular acceleration) at the same time, has a high difficulty, but is the most realistic form of the rehabilitation exercise. For example, if the eye target moves to the left while the head moves to the right, an eye movement system has to be activated in addition to the VOR, so a weighting effect of the two functions can be achieved. Conversely, if the eye target also moves to the right while the head moves to the right, the VOR that moves the eye in the same direction with respect to the head rotation has to be suppressed. Therefore, although it looks simple, the two configurations are methods of assessing and rehabilitating physiologically different functions.

Accordingly, in the present invention, the head and the eye are moved at the same time via the VOR function assessment unit 121, and the VOR function is assessed by comparing and analyzing the movement patterns thereof at this time.

When the rehabilitation exercise is requested, the patient-personalization control unit 122 determines, on the basis of the VOR gain of the patient, the type and order of rehabilitation games, and the rehabilitation level of the patient. In addition, while the rehabilitation exercise is being performed, the type and order of the rehabilitation game, and the rehabilitation level are adjusted frequently in consideration of the game result of the rehabilitation game providing unit 123.

The rehabilitation game providing unit 123 determines the type and order of the rehabilitation game to be provided to the patient according to the type and the order of the rehabilitation game determined by the patient-personalization control unit 122, and adjusts the game difficulty according to the rehabilitation level. At this time, the game difficulty is determined by at least one of the movement range and the movement speed (that is, at least one of the angular velocity and the angular acceleration) of the screen including the head rotation and the eye target. The movement range and the movement speed of the eye target or the background screen are increased in proportion to the game difficulty. In other words, it is possible to identify a condition of the patient based on the VOR gain and the rehabilitation exercise history of each patient, and configure and provide a customized rehabilitation game optimized for the condition of the patient based thereon.

For reference, the vestibular rehabilitation exercise includes a smooth pursuit which gently sees at a moving target, a saccade which moves the eye rapidly from one place to another, a VOR rehabilitation exercise (VOR) which induces the eye movement to a specific target point by inducing the eye movement with a proportional size and speed in the opposite direction to the head rotation, and the like.

In addition, in order to increase the effect of the VOR rehabilitation exercise, there are provided an amplified VOR rehabilitation exercise (augmentation VOR rehabilitation mode) which rotates the eye target more than the head rotation in a size or speed in the opposite direction of the rotation when the head rotation occurs, and a suppressive VOR rehabilitation exercise (suppression VOR rehabilitation mode) which conversely limits the eye target movement in the opposite direction to the rotation when the head rotation occurs in order to stably see an object moving in the same direction as the head rotation.

Accordingly, in the present invention, games for at least one of the smooth pursuit, the saccade, the VOR rehabilitation exercise, the amplified VOR rehabilitation exercise, and suppressive VOR rehabilitation exercise can be selectively configured and provided according to the VOR function and the purpose of the patient.

In addition, the rehabilitation game providing unit 123 monitors the concentration level of the patient, and when the concentration level falls to a preset value or more, it is possible to additionally generate and provide attentional information.

At this time, the concentration of the patient can be inferred based on an eye target watching frequency, a watching time, and an eye closing pattern sensed via the eye tracker 112, or inferred from a change pattern of the game score (that is, a sharp drop in the game score). In addition, the information for evoking attention can be implemented and provided by a method of changing the size, color, and shape of the eye target and the background screen (for example, a changing into a scary image or a picture of an acquaintance), or a sound stimulation method.

The communication unit 124 supports communication with an external device to provide information acquired via the control device 120 to the external device, or to receive and store information provided by the external device. In particular, it is possible to receive, install, or update program information for the VOR function assessment or the rehabilitation from the external device.

The operation panel 130 includes a keyboard, a mouse, buttons, a speaker, a monitor, and the like, and can receive various control values through them, or audiovisually guide a current driving state of the control device 120.

As described above, the present invention can increase the accuracy and efficiency of the rehabilitation exercise and realize reduction of manpower for the rehabilitation program.

FIG. 2 is a diagram illustrating a detailed configuration of the biosignal sensing unit according to an embodiment of the present invention.

Referring to FIG. 2, the biosignal sensing unit 110 includes an inertial measurement sensor 111, an eye tracker 112, and a synchronization unit 113.

The inertial measurement sensor 111 is implemented via at least one of an angular velocity sensor, an acceleration sensor, a gravity sensor, and a compass sensor, and senses the head movement of the user in three dimensions and performs the notification thereof.

The eye tracker 112 senses the eye movement (that is, gaze) based on the iris or pupil, and performs the notification thereof.

The synchronization unit 113 synchronizes the head movement and the eye movement of the user based on time to output them. This is to allow a comparison between a degree of the head movement and a degree of the eye movement of the user on the same time basis.

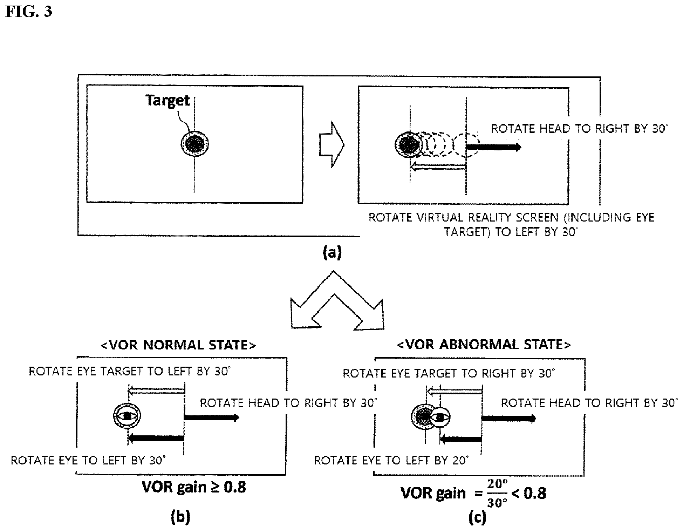

FIG. 3 is a diagram for explaining a VOR function assessment method according to an embodiment of the present invention.

If the patient or medical staff requests the VOR function assessment via the operation panel 130, the VOR function assessment unit 121 induces the head rotation at a preset angle and speed (acceleration) as illustrated in (a) of FIG. 3, configures and provides a game screen in which the entire virtual reality (VR) screen including the eye target and the background screen rotates in the opposite direction to the head rotation to induce the head rotation in various directions and speeds in a state where the patient fixes the his/her eye on the eye target.

When the VOR is in a normal state, the patient continuously see the eye target at the center of the screen while the eye rotates in the same direction and size in the opposite direction to the direction of head rotation as illustrated in (b) of FIG. 3.

However, as illustrated in (c) of FIG. 3, if the VOR is in an abnormal state, the patient cannot rotate the eye as much as the head movement angle so that a predetermined deviation occurs between the eye movement angle and the head movement angle.

Therefore, in the present invention, the movement angle and movement speed of the head and the eye are measured by using the inertial measurement sensor 111 and the eye tracker 112, and whether the VOR function is abnormal and furthermore, the degree of abnormality of the VOR function are identified based on the head-to-eye movement ratio (that is, the VOR gain). In particular, in the present invention, a more detailed VOR gain calculation operation can be performed by identifying the VOR gain by dividing the movement speed into the angular velocity and the angular acceleration.

[Equation 1]

$$positional\ VOR\ gain = \frac{eye\ rotation\ degree}{head\ rotation\ degree}$$

$$velocity\ VOR\ gain = \frac{eye\ rotation\ angular\ velocity}{head\ rotation\ angular\ velocity}$$

$$acceleration\ VOR\ gain = \frac{eye\ rotation\ angular\ acceleration}{head\ rotation\ angular\ acceleration}$$

That is, it is confirmed and notified that if the VOR gain is close to "1" (for example, 0.8 or more), the VOR is in the normal state, and as the VOR gain is closer to "0", the degree of VOR abnormality is more serious.

In addition, if the rotational movement is performed in the left and right directions, respectively, the gain is measured close to normal on the side having the normal VOR function, but if an abnormality occurs in the VOR function, the gain is reduced. Therefore, by comparing the gains according to the left and right rotational movements, the asymmetry of vestibulo-ocular reflex function can be calculated, and based on this, a lesion side with the VOR abnormality can be distinguished. This is briefly summarized in the equation as follows.

[Equation 2]

$$asymmetry(\%) = \frac{(right\ rotation\ gain) - (left\ rotation\ gain)}{(right\ rotaion\ gain) + (left\ rotation\ gain)} x100$$

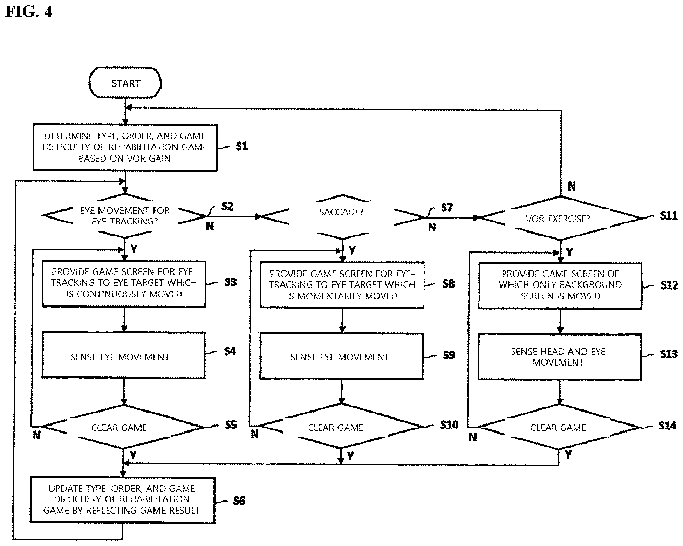

FIG. 4 is a flowchart for explaining a rehabilitation exercise supporting method according to an embodiment of the present invention.

First, the type, order, and game difficulty of the rehabilitation game to be provided to the patient are determined based on the VOR gain. That is, the condition of the patient is inferred based on the VOR gain, and a rehabilitation schedule optimized for the condition of the patient is determined and stored (S1).

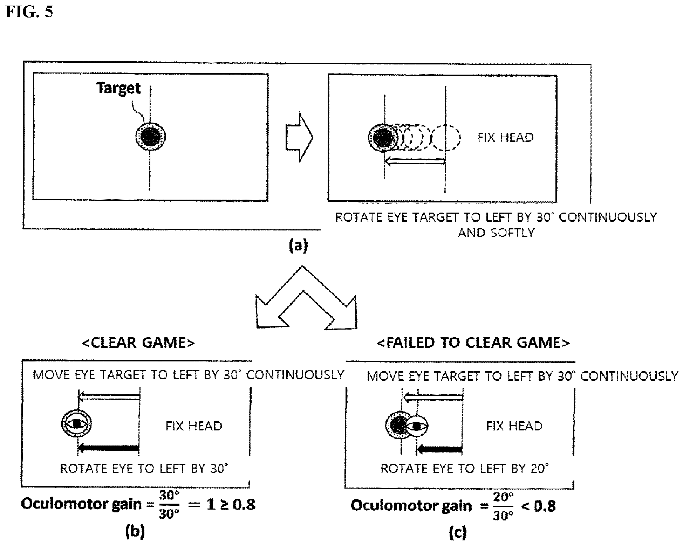

If the current game to be provided to the patient is a game for the smooth pursuit (S2), as illustrated in FIG. 5, a game screen, in which the eye target moves smoothly at a set speed, is configured and provided, and thereby the rehabilitation game providing unit 123 induces for the patient to continuously perform the eye track to the eye target (S3).

Then, the eye movement angle is sensed by using the eye tracker 112 (S4).

Then, by comparing the eye movement angle with the eye target movement angle, the ratio of the eye movement (oculomotor gain) to the eye movement target is calculated, and then the ratio is compared with a preset game target value (for example, 0.8) to determine whether the game is cleared (S5).

The game is repeatedly provided until the patient clears the game, and when the patient clears the game, the rehabilitation schedule is updated by reflecting the game result, and then the game to be provided next is confirmed (S6).

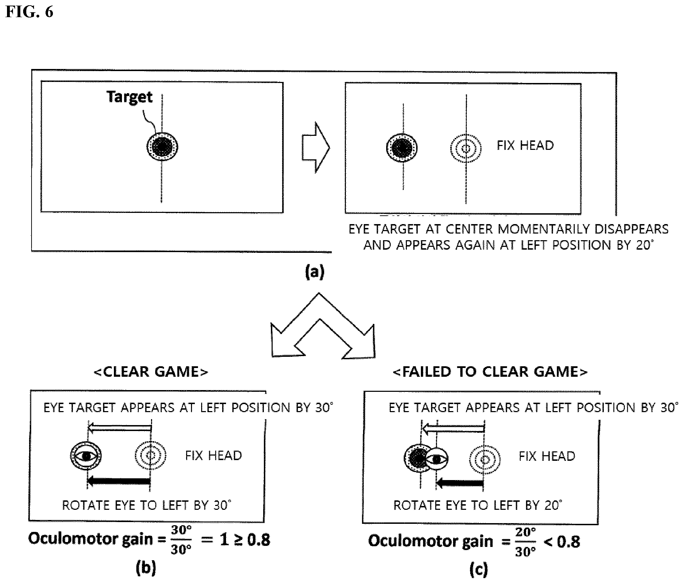

Accordingly, if the game to be provided to the patient is a game for the saccade (S7), as illustrated in FIG. 6, a game screen, in which the eye target appears at another position when the eye target disappears from the existing position, is configured and provided, and thereby the rehabilitation game providing unit 123 induces for the patient to accurately see each eye target in turn (S8).

Then, the eye movement angle is sensed by using the eye tracker 112 (S9) and the eye movement angle is compared and analyzed with the eye target movement angle to calculate the ratio of the eye movement (oculomotor gain) to the eye target. Then, it is determined whether the game is cleared by comparing the ratio of the eye movement (oculomotor gain) to the eye target, with a preset game target value (for example, 0.9) (S10).

[Equation 3]

$$positional\ oculomotor\ gain = \frac{eye\ rotation\ degree}{eye\ target\ rotation\ degree}$$

$$velocity\ oculomotor\ gain = \frac{eye\ rotation\ angular\ velocity}{eye\ target\ rotation\ angular\ velocity}$$

$$acceleration\ oculomotor\ gain = \frac{eye\ rotation\ angular\ acceleration}{eye\ target\ rotation\ angular\ acceleration}$$

When the patient clears the game, the process enters step S6, the rehabilitation schedule is updated by reflecting the game progress result, and then proceeds to the next game (S6).

Accordingly, if the game to be provided to the patient is a game for the VOR rehabilitation exercise (S11), the rehabilitation game providing unit 123 configures and provides a game screen in which the eye target rotates the head at a preset angle and speed, so that the patient fixes his/her eye to the eye target and induces the head to rotate (S12).

In addition, the head movement angle and the eye movement angle are simultaneously sensed by using the inertial measurement sensor 111 and the eye tracker 112 (S13), and then the ratio of the eye movement to the head is calculated by comparing and analyzing them. Then, it is determined whether the game is cleared by comparing the ratio of the eye movement to the head, with a preset game target value (for example, 0.8) (S14).

Accordingly, when the patient clears the game, the process enters step S6 and the rehabilitation schedule is updated by reflecting the game progress result, and when all the games are cleared, the rehabilitation level is increased by one step (S6). That is, in addition to the VOR gain, the rehabilitation level can also be determined in consideration of the rehabilitation exercise history.

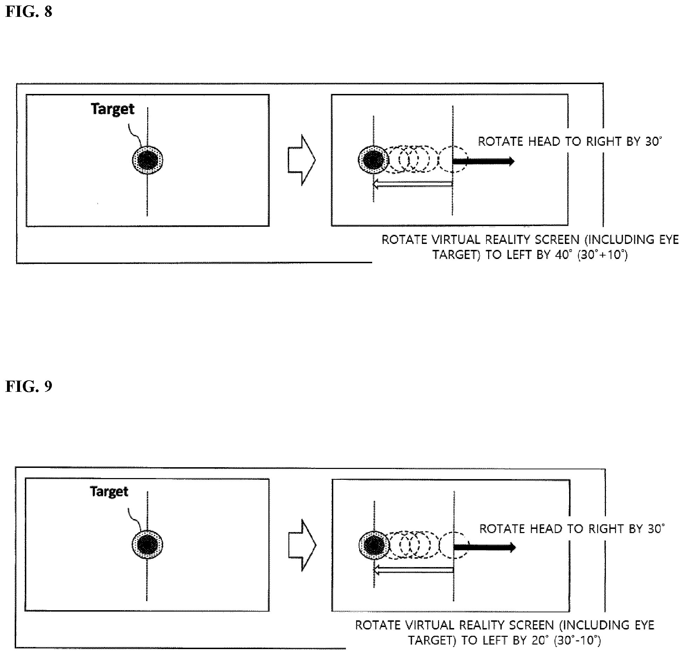

In addition, as illustrated in FIGS. 8 and 9, the rehabilitation exercise support method of the present invention can additionally configure and provide a game for the amplified VOR rehabilitation exercise (augmentation VOR rehabilitation mode) and the suppressive VOR rehabilitation exercise (suppression VOR rehabilitation mode).

First, as illustrated in FIG. 8, for the amplified VOR rehabilitation exercise (augmentation VOR rehabilitation mode), the game screen is configured and provided in the same principle as the VOR rehabilitation exercise, but the movement degree of the eye target is amplified and provided.

In other words, a game screen, in which the eye target is rotated more than the VOR rehabilitation exercise at a constant ratio (1.1 times, 1.2 times, 1.5 times, or the like) in the opposite direction to the head rotation, is configured and provided. By measuring and analyzing the eye and head movements at this time, the VOR exercise and eye movement functions can be assessed and rehabilitated.

In addition, as illustrated in FIG. 9, for the suppressive VOR rehabilitation exercise (suppression VOR rehabilitation mode), the game screen is configured and provided in the same principle as the VOR rehabilitation exercise, but the movement degree of the eye target is suppressed and provided.

That is, a game screen, in which the size or speed at which the eye target rotates in the opposite direction to the head rotation are decreased by a certain ratio (0.9 times, 0.8 times, 0.5 times, or the like), is configured and provided. By measuring and analyzing the eye and head movements at this time, the VOR exercise and the eye movement function can be assessed and rehabilitated.

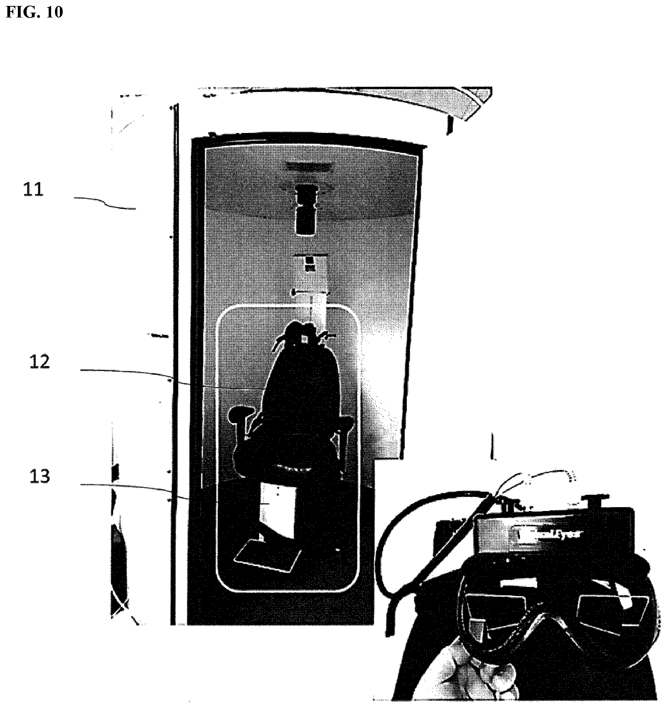

On the other hand, as illustrated in FIG. 10, the vestibulo-ocular reflex assessment operation can be performed in a VOR function assessing method in which the testee is seated on a swivel chair provided in a darkroom, and then the body of the testee is directly rotated via the chair while light that rotates is provided in which light-dark is repeated in a shape of a railroad (rail road stripe) (VVOR) or a laser that rotates according to the rotation of the chair is provided.

However, the method has a problem in that the darkroom and the swivel chair are required to secure a dark field, so it takes a lot of time and money to construct the system, and it is also very difficult to move the test site.

Accordingly, in the present invention, a new method for performing the vestibulo-ocular reflex assessment operation at anytime and anywhere by using all of the existing swivel chairs without requiring a separate darkroom as illustrated in FIG. 10 is additionally proposed.

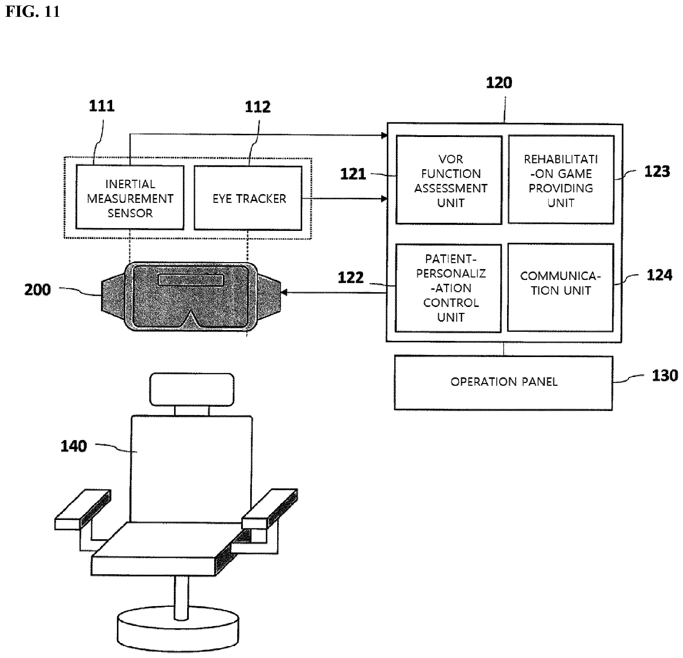

FIG. 11 is a diagram illustrating a virtual reality-based vestibulo-ocular reflex assessment and rehabilitation apparatus according to another embodiment of the present invention.

As illustrated in FIG. 11, the vestibulo-ocular reflex assessment and rehabilitation apparatus of the present invention can further include a swivel chair 140 in addition to the configuration elements 110, 120, and 130 of FIG. 1.

The swivel chair 140 is a device that supports the seating and rotation of the testee, and can be implemented by any rotatable general chair. That is, the present invention can utilize all of the ready-made chairs instead of the conventional self-produced chairs.

In addition, the swivel chair 140 can be rotated manually by the tester to induce the head of the testee to rotate (manual rotation), or the testee can rotate his/her head according to a guide displayed on a screen through the head-mounted display device 200 (active rotation). Of course, if necessary, the swivel chair 140 can be implemented by a method of automatically rotating a seat plate and a backrest at a preset rotation angle and speed based on a motor under the control of the control device 120.

That is, the present invention is not a method of making a preset rotation pattern through a conventional self-produced chair, but a method of measuring the rotation pattern through the inertial measurement sensor 111 to perform the vestibulo-ocular reflex assessment operation by using all the general swivel chairs. As a result, it is possible to secure portability of the apparatus and simplify the inspection method by minimizing the apparatus volume and implementation cost.

However, it is preferable that the head-mounted display device 200 is in perfect contact with the eye region of the testee, so that the vestibulo-ocular reflex assessment and rehabilitation operation can be performed in the dark field state by blocking external light.

Then, the VOR function assessment unit 121 in the control device 120 generates a test screen for inducing a visual stimulus (eye rotation) in association with the head rotation to reproduce it via the head-mounted display device 120. At the same time, based on the sensing results of the eye tracker 112 and the inertial measurement sensor 111, the ratio of the eye rotation to the head rotation is calculated and quantified to obtain and provide the VOR gain, the asymmetry ratio, the visual vestibulo-interaction, and the eye fixation effect.

As described above, in the present invention, by sensing the head rotation and the eye rotation by using the inertial measurement sensor and the eye tracker, and temporally integrating and analyzing information obtained from these two devices, an appropriate vestibulo-ocular reflex for the head rotation stimulus is obtained to confirm whether the eye movement in the opposite direction occurs in real time at an appropriate angle, angular velocity, and angular acceleration.

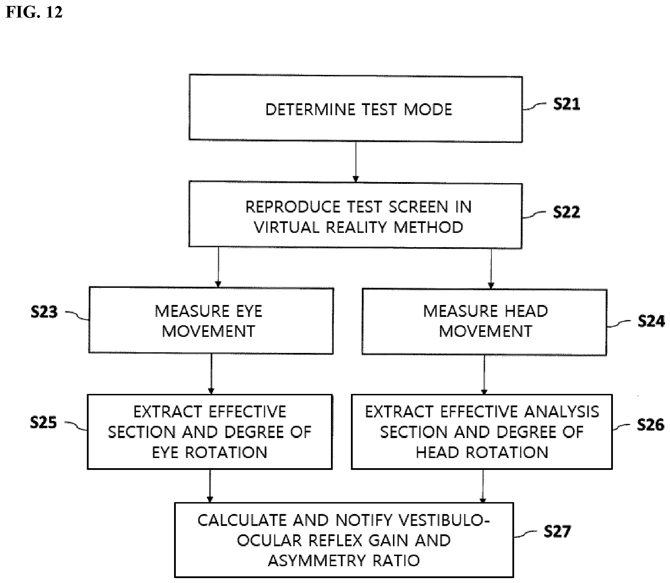

FIG. 12 is a flowchart for explaining a vestibulo-ocular reflex function assessment method according to another embodiment of the present invention.

First, when a test mode is determined at the request of the testee or the tester (S21), the VOR function assessment unit 121 configures a test screen corresponding to the test mode, and then uses the virtual reality method via the head-mounted display device 120 to reproduce the test screen (S22).

The test mode of the present invention can be divided into a basic vestibulo-ocular reflex (VOR) test mode, a visual vestibulo-ocular reflex (VVOR) test mode, and a visual fixation effect (VFX) test mode.

In addition, in the basic vestibulo-ocular reflex (VOR) test mode, the virtual reality screen is basically configured to be darkened and a screen that does not display any eye points capable of being fixed for eye is configured to enable pure vestibulo-ocular reflex measurement. That is, it configures and provides a test screen for providing the dark field, and enables the vestibulo-ocular reflex measurement in the corresponding state.

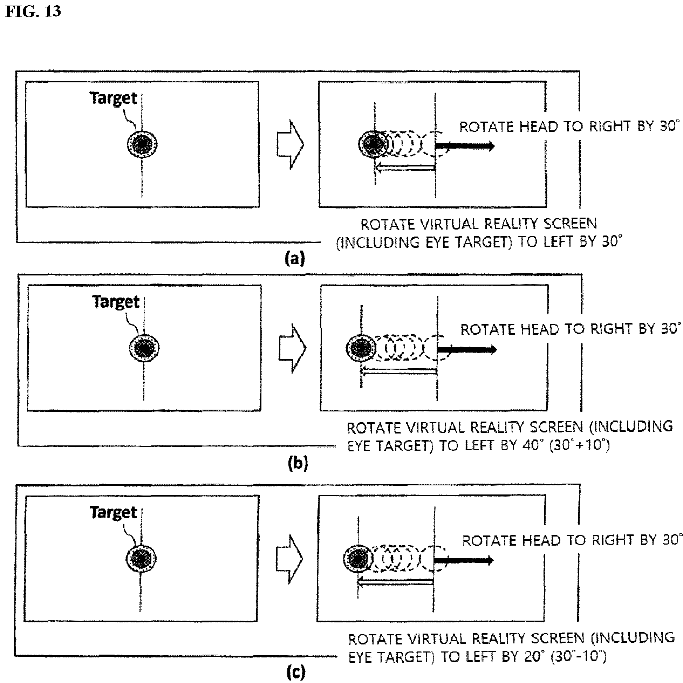

In addition, in the visual vestibulo-ocular reflex (VVOR) test mode, as illustrated in (a) and (b) of FIG. 13, which illustrate the situation in the real world under the virtual reality screen, a test screen in which the eye rotation compared to the head rotation can be adjusted at various ratios is configured and provided. Therefore, the testee generates an amplified eye rotation to the head rotation. In particular, in addition to setting the degree of the head rotation and the degree of the eye rotation to be the same as illustrated in (a) of FIG. 13, it is possible to increase the degree of the eye rotation compared to the degree of the head rotation as illustrated in (b) of FIG. 13. This corresponds to the augmentation visual-vestibular interaction test mode. In the real world, for example, it is possible to assess the ability to stably see a car moving in the same direction as the head rotation.

Finally, in the visual fixation effect (VFX) test mode, the visual objective rotates at the same angular velocity as the head rotation in the previous inspection, compared to the existing test where only the full visual effect could be assessed by rotating the eye target at the same angular velocity as the head rotation in the existing inspection, as illustrated in (c) of FIG. 13, a test screen, in which the ratio of the eye rotation to the head rotation can be suppressed at various ratios, is configured and provided. Therefore, the testee generates a suppressed eye rotation to the head rotation. This is the suppression visual-vestibular interaction test mode. In the real world, for example, it is possible to assess the ability to stably see a car moving in the opposite direction to the head rotation.

Through step S21, the test screen is reproduced in the virtual reality method, and when the testee moves the eye and the head in response thereto, the eye and head rotations are immediately measured by using the eye tracker 112 and the inertial measurement sensor 111 (S23).

After the head rotation data undergoes a process of removing measurement noise thereof, precise estimation values of a rotation amount and rotation characteristics are acquired through artificial intelligence (AI)-based machine learning. Then, a frequency characteristic is analyzed by performing a Fast Fourier transform on the acquired estimation values, and then the appropriateness of the measured value compared to the target frequency is assessed by comparing the measured value with the target test frequency. If the head rotation is inappropriate, a retry of the head rotation is requested, and then the same test screen is reproduced again. On the other hand, in a case where a proper head rotation is made, an effective section in which the rotation in the target range is made and the degree of the head rotation (rotation angle, angular velocity, and angular acceleration) are extracted (S24).

At the same time, after noises such as spontaneous eye movement, nystagmus, and eye blinking from the eye movement data are removed, AI-based machine learning is performed to acquire a precise estimation value of the eye rotation. Then, the frequency characteristic is analyzed by performing the Fast Fourier transform on the acquired estimation value, and the degree of the eye rotation (rotation angle, angular velocity, and angular acceleration) in an appropriate rotation section and frequency is extracted (S25).

In addition, after the degree of the eye rotation (angle, angular velocity, and angular acceleration) compared to the degree of the head rotation (angle, angular velocity, and angular acceleration) is calculated according to the following equation, the VOR gain including at least one of them, and an asymmetry ratio thereof are acquired and provided (S26).

That is, a rotation angle-based gain is calculated from the eye rotation angle to the head rotation angle according to Equation 4, and the maximum rotation angular velocity-based gain is calculated from the maximum eye rotation angular velocity to the maximum head rotation angular velocity according to Equation 5. After the maximum rotation angular acceleration-based gain is calculated from the maximum eye rotation angular acceleration to the maximum head rotation angular acceleration according to Equation 6, the VOR gain including at least one of the rotation angle-based gain, the maximum rotation angular velocity-based gain, and the maximum rotation angular acceleration-based gain is generated and output.

[Equation 4]

$$positional\ gain = \frac{eye\ rotation\ degree\ of\ effective\ section}{head\ rotation\ degree\ of\ effective\ section}$$

[Equation 5]

$$velocity\ gain = \frac{eye\ rotation\ angular\ velocity\ of\ effective\ section}{head\ rotation\ angular\ velocity\ of\ effective\ section}$$

[Equation 6]

$$acceleration\ gain = \frac{maximum\ eye\ rotation\ angular\ acceleration\ of\ effective\ section}{maximum\ head\ rotation\ angular\ acceleration\ of\ effective\ section}$$

In this case, the effective section means a section in which the rotation of the target range identified through the machine learning and Fourier transform-based analysis is made.

In addition, the asymmetry ratio can be acquired according to Equation 7 below.

[Equation 7]

$$asymmetry(\%) = \frac{(\text{degree of rotation to right}) - (\text{degree of rotation to left})}{(\text{degree of rotation to right}) + (\text{degree of rotation to left})} \times 100$$

In this case, the degree of the rotation is any one of the rotation angle, the angular velocity, and the angular acceleration.

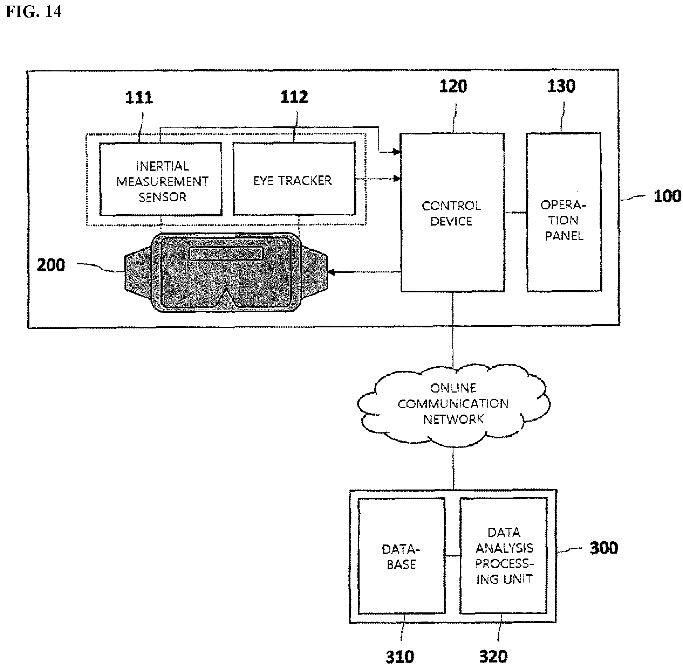

FIG. 14 is a diagram illustrating a virtual reality-based vestibulo-ocular reflex assessment and rehabilitation system according to another embodiment of the present invention.

As illustrated in Figure 14, the system of the present invention is configured of a plurality of vestibulo-ocular reflex assessment and rehabilitation apparatuses 100 and a central control server 300 that interoperates with each of the plurality of vestibulo-ocular reflex assessment and rehabilitation apparatuses 100, so that various functions that have been distributed and executed in each of the vestibulo-ocular reflex assessment and rehabilitation apparatuses 100 can be integrated and performed on the server side.

In this case, the vestibulo-ocular reflex assessment and rehabilitation apparatus 100 and the central control server 300 can be connected via a network in a cloud manner, or the like. This is for the vestibulo-ocular reflex assessment and rehabilitation apparatus 100 to be provided to interoperate with the central control server 300 regardless of the location and time thereof.

The central control server 300 is configured of a database 310 and a data analysis processing unit 320, and the database 310 divides information provided by each of the vestibulo-ocular reflex assessment and rehabilitation apparatuses 100 for each patient account to store and manage the information. The data analysis processing unit 320 acquires and analyzes the patient information stored in the database 310 to acquire the patient test result (that is, VOR gain and asymmetry ratio, visual-vestibular interaction, and fixation effect), or to configure and provide a patient-personalization rehabilitation program corresponding to the patient test result. At this time, the patient-personalization rehabilitation program performs at least one of the smooth pursuit, the saccade, the VOR rehabilitation exercise, the amplified VOR rehabilitation exercise, and the suppressive VOR rehabilitation exercise in the virtual reality game method.

In particular, the data analysis processing unit 320 employs a high-performance, large-capacity processor, so that much data can be analyzed and processed more quickly and accurately, and furthermore, data analysis and processing can be performed in a neural network-based machine learning method.

Accordingly, the vestibulo-ocular reflex assessment and rehabilitation apparatus 100 can be implemented to have only a minimum configuration and function. For example, the vestibulo-ocular reflex assessment and rehabilitation apparatus 100 simply reproduces a screen for the VOR function assessment or the rehabilitation exercise, performs only a basic function of sensing and outputting only the head and eye movements of the patient at this time, and allows the central control server 300 to perform all of the remaining functions such as analysis and processing of sensing information.

That is, in the present invention, the configuration of the vestibulo-ocular reflex assessment and rehabilitation apparatus 100 is minimized, and the main functions thereof are integrated and performed in the central control server 300, thereby expanding system accessibility and reducing system construction costs to be minimized.

In addition, as the patient information is integrated and managed in the central control server 300, the continuity of the patient assessment and the rehabilitation operation can be ensured even in a case where the device used by the patient and the medical staff in charge of the patient are changed.

The above description is merely illustrative of the technical spirit of the present invention and various modifications and variations will be possible without departing from the essential characteristics of the present invention by those skilled in the art to which the present invention pertains. Therefore, the embodiments disclosed in the present invention are not intended to limit the technical spirit of the present invention, but to explain the technical spirit thereof, and the scope of the technical spirit of the present invention is not limited by these embodiments. The protection scope of the present invention should be interpreted by the following claims, and all technical ideas within the scope equivalent thereto should be construed as being included in the scope of the present invention.

**Claims**

1. A virtual reality game- and biosignal sensor-based vestibulo-ocular reflex assessment and rehabilitation apparatus, comprising:

a biosignal sensing unit which senses and notifies of head movement and eye movement of a patient using an eye tracker and an inertial measurement unit (IMU) sensor attached or installed to a head-mounted display device;

a VOR function assessment unit which, when a VOR function assessment is requested, configures and provides a game for VOR function assessment, and then obtains a VOR gain by calculating and quantifying the ratio of eye to head movement from the sensing result of the IMU sensor and the eye tracker;

a patient-personalization control unit which, when rehabilitation exercise is requested, determines, on the basis of the VOR gain of the patient, the types and order of rehabilitation games, and a rehabilitation level; and

a rehabilitation game providing unit which, according to the types and order of the rehabilitation games, configures and provides games for at least one of a smooth pursuit, a saccade, a VOR rehabilitation exercise, an amplified VOR rehabilitation exercise, and a suppressive VOR rehabilitation exercise, and determines the difficulty levels of the games on the basis of the rehabilitation level.

2. The virtual reality game- and biosignal sensor-based vestibulo-ocular reflex assessment and rehabilitation apparatus according to claim 1, wherein the patient-personalization control unit further includes a function of frequently adjusting the type and order of the rehabilitation game, and the rehabilitation level thereof in consideration of the game result of the rehabilitation game providing unit while the rehabilitation exercise is being performed.

3. The virtual reality game- and biosignal sensor-based vestibulo-ocular reflex assessment and rehabilitation apparatus according to claim 1, wherein the VOR function assessment unit configures and provides a game in which the eye observes the eye target on the screen and which induces a head rotation at a preset angle and velocity to induce the VOR.

4. The virtual reality game- and biosignal sensor-based vestibulo-ocular reflex assessment and rehabilitation apparatus according to claim 1, wherein the rehabilitation game providing unit configures and provides a game for eye-tracking an eye target in which the eye moves smoothly at a set speed when the smooth pursuit is requested, and then calculates and analyzes a ratio of the eye movement to the eye target to determine whether the game is cleared.

5. The virtual reality game- and biosignal sensor-based vestibulo-ocular reflex assessment and rehabilitation apparatus according to claim 1, wherein the rehabilitation game providing unit configures and provides a game for eye-tracking an eye target in which the eye momentarily moves from one place to another when the saccade is requested, and then calculates and analyzes a ratio of the eye movement to the eye target to determine whether the game is cleared.

6. The virtual reality game- and biosignal sensor-based vestibulo-ocular reflex assessment and rehabilitation apparatus according to claim 1, wherein the rehabilitation game providing unit configures and provides a game for rotating only the head at a preset angle and speed while keeping the eye fixed when the VOR rehabilitation exercise is requested, and then calculates and analyzes a ratio of the eye movement to the eye target to determine whether the game is cleared.

7. The virtual reality game- and biosignal sensor-based vestibulo-ocular reflex assessment and rehabilitation apparatus according to claim 1, wherein the rehabilitation game providing unit configures and provides a game for inducing the head movement at a preset angle and speed, and further rotating the eye target at a ratio or more to the head rotation to induce a greater range of the eye movement when the amplified VOR rehabilitation exercise is requested, and then calculates and analyzes a ratio of the eye movement to the eye target to determine whether the game is cleared.

8. The virtual reality game- and biosignal sensor-based vestibulo-ocular reflex assessment and rehabilitation apparatus according to claim 1, wherein the rehabilitation game providing unit configures and provides a game for inducing the head movement at a preset angle and speed, and inducing the eye movement for suppressing the eye target to be rotated less at a certain ratio to the head rotation when the suppressive VOR rehabilitation exercise is requested, and then calculates and analyzes a ratio of the eye movement to the eye target to determine whether the game is cleared.

9. The virtual reality game- and biosignal sensor-based vestibulo-ocular reflex assessment and rehabilitation apparatus according to claim 1, wherein the rehabilitation game providing unit further includes a function of monitoring the concentration of the patient, and additionally generating and providing information for evoking attention when the concentration falls to a preset value or more.

**10.** The virtual reality game- and biosignal sensor-based vestibulo-ocular reflex assessment and rehabilitation apparatus according to claim 1, wherein the rehabilitation game providing unit adjusts a game difficulty through at least one of the movement speed of the eye target, the rotation speed of the head, and the movement speed of the background screen.

**11.** The virtual reality game- and biosignal sensor-based vestibulo-ocular reflex assessment and rehabilitation apparatus according to claim 1, further comprising:
a swivel chair that supports seating and rotation of a testee.

**12.** The virtual reality game- and biosignal sensor-based vestibulo-ocular reflex assessment and rehabilitation apparatus according to claim 11, wherein the VOR function assessment unit further includes a function of generating a test screen for providing a dark field to reproduce the test screen via the head-mounted display device, and then calculating and quantifying the ratio of the eye rotation to the head rotation from the sensing results of the eye tracker and the inertial measurement sensor to calculate and provide at least one of the VOR (Vestibulo-Ocular Reflex) gain and the asymmetry ratio.

**13.** The virtual reality game- and biosignal sensor-based vestibulo-ocular reflex assessment and rehabilitation apparatus according to claim 11, wherein the VOR function assessment unit further includes a function of generating and providing a test screen inducing preset head rotation and eye rotation, and then calculating and quantifying the ratio of the eye rotation to the head rotation from the sensing results of the eye tracker and the inertial measurement sensor to calculate and provide at least one of the VOR (Vestibulo-Ocular Reflex) gain and the asymmetry ratio.

**14.** The virtual reality game- and biosignal sensor-based vestibulo-ocular reflex assessment and rehabilitation apparatus according to claim 11, wherein the VOR function assessment unit configures and provides a test screen inducing preset head rotation and eye rotation, and adjusts, in multiple steps, the ratio of the eye rotation to the head rotation, the rotation angles and speeds of the head and the eye.

**15.** The virtual reality game- and biosignal sensor-based vestibulo-ocular reflex assessment and rehabilitation apparatus according to claim 11, wherein the VOR function assessment unit calculates the rotation angle-based gain from a ratio of the eye rotation to the head rotation angle, calculates a maximum rotation angular velocity-based gain from a maximum eye rotation angular velocity to a maximum head rotation angular velocity, calculates a maximum rotation angular acceleration-based gain from a maximum eye rotation angular acceleration to a maximum head rotation angular acceleration, and then selects and provides, as the VOR gain, at least one of the rotation angle-based gain, the maximum rotation angular velocity-based gain, and the maximum rotation angular acceleration-based gain.

**16.** The virtual reality game- and biosignal sensor-based vestibulo-ocular reflex assessment and rehabilitation apparatus according to claim 11, wherein the VOR function assessment unit calculates the asymmetry ratio according to the expression "(((degree of rotation to right)-(degree of rotation to left))/((degree of rotation to right)+(degree of rotation to left)))x100", and the degree of the rotation is any one of the rotation angle, the rotation angular velocity, and the rotation angular acceleration.

**17.** The virtual reality game- and biosignal sensor-based vestibulo-ocular reflex assessment and rehabilitation apparatus according to claim 11, wherein the VOR function assessment unit requests a retry of the head rotation, and then performs the vestibulo-ocular reflex function assessment operation once more when the head rotation is not within a preset target frequency.

**18.** The virtual reality game- and biosignal sensor-based vestibulo-ocular reflex assessment and rehabilitation apparatus according to claim 11, wherein the swivel chair is automatically rotated under the control of the VOR function assessment unit or manually rotated by the testee or the tester.

**19.** A virtual reality-based vestibulo-ocular reflex assessment and rehabilitation system comprising:

a plurality of vestibulo-ocular reflex assessment and rehabilitation apparatuses which reproduce a screen for a VOR (Vestibulo-Ocular Reflex) function assessment or a rehabilitation exercise, use an eye tracker and an inertial measurement sensor (IMU sensor) attached or installed on a head-mounted display device to sense and output a head movement and an eye movement of a patient; and
a central control server which divides information provided by each of the vestibulo-ocular reflex assessment and rehabilitation apparatuses for each patient account to store the information in a database, and acquires and analyzes the patient information stored in the database to assess a VOR gain, or configures and provides a patient-personalization rehabilitation program based on the VOR gain,

wherein in the patient-personalization rehabilitation program, at least one of a smooth pursuit, a saccade, a VOR rehabilitation exercise, an amplified VOR rehabilitation exercise, and a suppressive VOR rehabilitation exercise is performed in a virtual reality game method.

**FIG. 1**

**111**  **112**  **120**

INERTIAL
MEASUREMENT
SENSOR

EYE
TRACKER

**121**
VOR FUNCTION
ASSESSMENT
UNIT

REHABILITATION
GAME
PROVIDING UNIT
**123**

**200**

**122**
PATIENT-
PERSONALIZATI-
ON CONTROL
UNIT

COMMUNICA
-TION UNIT
**124**

OPERATION PANEL
**130**

**FIG. 2**

**111**
INERTIAL
MEASUREMENT
SENSOR

SYNCHRONIZATION
UNIT
**113**

**112**
EYE TRACKER

**FIG. 3**

ROTATE HEAD TO RIGHT BY 30°

ROTATE VIRTUAL REALITY SCREEN (INCLUDING EYE TARGET) TO LEFT BY 30°

**(a)**

**<VOR NORMAL STATE>**

ROTATE EYE TARGET TO LEFT BY 30°

ROTATE HEAD TO RIGHT BY 30°

ROTATE EYE TO LEFT BY 30°

**VOR gain ≥ 0.8**

**(b)**

**<VOR ABNORMAL STATE>**

ROTATE EYE TARGET TO RIGHT BY 30°

ROTATE HEAD TO RIGHT BY 30°

ROTATE EYE TO LEFT BY 20°

$$\text{VOR gain} = \frac{20°}{30°} < 0.8$$

**(c)**

**FIG. 4**

FIG. 5

ROTATE EYE TARGET TO LEFT BY 30° CONTINUOUSLY
AND SOFTLY

(a)

<CLEAR GAME>                                          <FAILED TO CLEAR GAME>

MOVE EYE TARGET TO LEFT BY 30° CONTINUOUSLY          MOVE EYE TARGET TO LEFT BY 30° CONTINUOUSLY

FIX HEAD                                              FIX HEAD

ROTATE EYE TO LEFT BY 30°                             ROTATE EYE TO LEFT BY 20°

$$\text{Oculomotor gain} = \frac{30°}{30°} = 1 \geq 0.8$$          $$\text{Oculomotor gain} = \frac{20°}{30°} < 0.8$$

(b)                                                  (c)

**FIG. 6**

EYE TARGET AT CENTER MOMENTARILY DISAPPEARS
AND APPEARS AGAIN AT LEFT POSITION BY 20°

**(a)**

**<CLEAR GAME>**

EYE TARGET APPEARS AT LEFT POSITION BY 30°

FIX HEAD

ROTATE EYE TO LEFT BY 30°

$$\text{Oculomotor gain} = \frac{30°}{30°} = 1 \geq 0.8$$

**(b)**

**<FAILED TO CLEAR GAME>**

EYE TARGET APPEARS AT LEFT POSITION BY 30°

FIX HEAD

ROTATE EYE TO LEFT BY 20°

$$\text{Oculomotor gain} = \frac{20°}{30°} < 0.8$$

**(c)**

**FIG. 7**

Target

ROTATE HEAD TO RIGHT BY 30°

ROTATE VIRTUAL REALITY SCREEN
(INCLUDING EYE TARGET) TO LEFT BY 30°

(a)

<CLEAR GAME>

ROTATE EYE TARGET TO LEFT BY 30°

ROTATE HEAD TO RIGHT BY 30°

ROTATE EYE TO LEFT BY 30°

$$\text{VOR gain} = \frac{30°}{30°} = 1 \geq 0.8$$

(b)

<FAILED TO CLEAR GAME>

ROTATE EYE TARGET TO LEFT BY 30°

ROTATE HEAD TO RIGHT BY 30°

ROTATE EYE TO LEFT BY 20°

$$\text{VOR gain} = \frac{20°}{30°} < 0.8$$

(c)

**FIG. 8**

Target

ROTATE HEAD TO RIGHT BY 30°

ROTATE VIRTUAL REALITY SCREEN (INCLUDING EYE
TARGET) TO LEFT BY 40° (30°+10°)

**FIG. 9**

Target

ROTATE HEAD TO RIGHT BY 30°

ROTATE VIRTUAL REALITY SCREEN (INCLUDING EYE
TARGET) TO LEFT BY 20° (30°-10°)

**FIG. 10**

**FIG. 11**

**FIG. 12**

| DETERMINE TEST MODE | S21 |

| REPRODUCE TEST SCREEN IN VIRTUAL REALITY METHOD | S22 |

S23 — | MEASURE EYE MOVEMENT | | MEASURE HEAD MOVEMENT | — S24

S25 — | EXTRACT EFFECTIVE SECTION AND DEGREE OF EYE ROTATION | | EXTRACT EFFECTIVE ANALYSIS SECTION AND DEGREE OF HEAD ROTATION | — S26

| CALCULATE AND NOTIFY VESTIBULO-OCULAR REFLEX GAIN AND ASYMMETRY RATIO | — S27

**FIG. 13**

Target

ROTATE HEAD TO RIGHT BY 30°

ROTATE VIRTUAL REALITY SCREEN (INCLUDING EYE TARGET) TO LEFT BY 30°

**(a)**

Target

ROTATE HEAD TO RIGHT BY 30°

ROTATE VIRTUAL REALITY SCREEN (INCLUDING EYE TARGET) TO LEFT BY 40° (30°+10°)

**(b)**

Target

ROTATE HEAD TO RIGHT BY 30°

ROTATE VIRTUAL REALITY SCREEN (INCLUDING EYE TARGET) TO LEFT BY 20° (30°-10°)

**(c)**

**FIG. 14**